# EUROPÄISCHE PATENTANMELDUNG

(11) **EP 4 224 382 A1**
(43) Veröffentlichungstag der Anmeldung: **09.08.2023**
(21) Anmeldenummer: 22155157.5
(22) Anmeldetag: 04.02.2022
(51) Int. Cl.: G06Q 10/00, G16H 40/40, G05B 23/02

(54) **TECHNIK ZUR DURCHSETZUNG EINER BETRIEBSSICHERHEIT**

(71) Anmelder: Siemens Healthcare GmbH, 91052 Erlangen (DE)
(72) Erfinder: Baumann, Berthold, 95506 Kastl (DE); Krämer, Alexander, 92699 Irchenrieth (DE); Schmidt, Verena, 92681 Erbendorf (DE); Wurzer, Gerhard, 92708 Mantel (DE); Zeidler, Josef, 95615 Marktredwitz (DE)

(57) **Zusammenfassung**

Die vorliegende Erfindung betrifft eine Technik zur Bestimmung und Durchsetzung einer Betriebssicherheit einer technischen Komponente (502), die in einem medizinischen Feldgerät verbaut ist. Dies erfolgt in Abhängigkeit von Sensordaten des medizinischen Feldgeräts, die über eine Gesamtbetriebszeit der technischen Komponente (502) mittels eines Sensors überwacht werden. Das Verfahren basiert auf einem Digitalen Zwilling (504) und/oder einem Betriebssicherheitsmodell (506) und einem in einer Verarbeitungseinheit (508) gespeicherten Betriebssicherheitszeitberechnungsalgorithmus. Als Zwischenergebnis wird eine berechnete Restbetriebslaufzeit der technischen Komponente (502) ausgegeben und ein Steuersignal zum Steuern des medizinischen Feldgerätes auf Basis der berechneten Restbetriebslaufzeit erzeugt.

## Beschreibung

Die vorliegende Erfindung betrifft eine Technik, insbesondere ein Verfahren, zur Bestimmung und Durchsetzung einer Betriebssicherheit einer technischen Komponente eines medizinischen Feldgeräts in Abhängigkeit von überwachten Sensordaten.

Medizinische Systeme werden herkömmlicherweise für eine Betriebszeit von mindestens zehn Jahren (Gewährleistung im Feld) bei maximaler Anzahl von zu behandelnden Patienten, maximaler Anzahl an Beschleunigungs- und Bremsvorgängen bzw. mittlerem bis maximal zu bewegendem Gewicht ausgelegt. Dies basiert auf einem sogenannten Betriebsfestigkeitsnachweis bzw. Betriebssicherheitsnachweis, der aus konservativen Schätzungen, basierend auf standardisierten Sicherheitstests, berechnet wird. Eine Vielzahl der Systeme wird während den zehn Jahren der herkömmlichen Betriebszeit allerdings mit weitaus weniger Lastzyklen belastet als in dem konservativen Betriebsfestigkeitsnachweis angenommen. Die Folge ist, dass ein großer Teil der im Feld vorhanden Systeme erheblich überdimensioniert ist. Eine kleinere Dimensionierung könnte einen Materialverbrauch und Produktkosten erheblich senken, bzw. bei gleicher Dimensionierung könnten die Systeme eine deutlich höhere Betriebszeit aufweisen als herkömmlicherweise konservativ angenommen. Herkömmlicherweise werden medizinische Systeme für eine stets maximale Belastung dimensioniert und nach der im Vorhinein für diese maximale Belastung vorgegebene Betriebszeit, beispielsweise nach zehn Jahren, komplett aufbereitet oder ausgetauscht, obwohl eine über die Betriebszeit akkumulierte maximale Belastung, die die Betriebssicherheit gefährden würde, noch nicht erreicht worden ist.

Der vorliegenden Erfindung liegt daher die Aufgabe zugrunde, eine Technik zur Bestimmung und Durchsetzung einer Betriebssicherheit einer technischen Komponente eines medizinischen Feldgeräts in Abhängigkeit von einer tatsächlichen Belastung, einer tatsächlichen Benutzung und/oder einem tatsächlichen Verschleiß zu bestimmen. Alternativ oder ergänzend liegt der Erfindung die Aufgabe zugrunde, eine Betriebsdauer zu verlängern und/oder eine leichtere Bauweise eines medizinischen Feldgeräts zu ermöglichen.

Diese Aufgabe wird jeweils durch einen Gegenstand nach den unabhängigen und nebengeordneten Ansprüchen gelöst. Vorteilhafte Ausführungsformen sind Gegenstand der abhängigen Ansprüche, der Beschreibung und der Zeichnungen.

Gemäß einem ersten Aspekt wird die Aufgabe gelöst durch ein Verfahren zur Bestimmung und Durchsetzung einer Betriebssicherheit einer technischen Komponente eines medizinischen Feldgerätes. Das Verfahren basiert auf Sensordaten, die während einer Gesamtbetriebszeit der technischen Komponente mittels mindestens eines Sensors überwacht werden. Das Verfahren umfasst einen Verfahrensschritt des Empfangens von Sensordaten mittels des mindestens einen Sensors während einer Betriebszeit der technischen Komponente des medizinischen Feldgeräts. Das Verfahren umfasst ferner einen Verfahrensschritt des Speicherns der mittels des mindestens einen Sensors empfangenen Sensordaten in einem Datenspeicher. Das Verfahren umfasst ferner einen Verfahrensschritt des Zugreifens auf eine Verarbeitungseinheit, in der ein Digitaler Zwilling und/oder ein Betriebssicherheitsmodell gespeichert ist. Das Zugreifen erfolgt mit den empfangenen Sensordaten, um auf Basis eines in der Verarbeitungseinheit gespeicherten Betriebssicherheitszeitberechnungsalgorithmus eine Restbetriebslaufzeit der technischen Komponente zu berechnen. Das Verfahren umfasst ferner einen Verfahrensschritt des Ausgebens der berechneten Restbetriebslaufzeit der technischen Komponente als Zwischenergebnis. Das Verfahren umfasst ferner einen Verfahrensschritt des Erzeugens mindestens eines Steuersignals zum Steuern des medizinischen Feldgerätes auf Basis der berechneten Restbetriebslaufzeit, um den Betrieb des medizinischen Feldgerätes einzuschränken, vollständig zu sperren oder mit mindestens einer veränderten Steuergröße fortzusetzen.

Der Digitale Zwilling und/oder das Betriebssicherheitsmodell kann als digitales Simulationsmodell ausgebildet sein. Alternativ oder ergänzend kann der Digitale Zwilling oder das Betriebssicherheitsmodell jeden Betrieb, z.B. in Form einer Bewegung und/oder Belastung der jeweiligen technischen Komponente und/oder des medizinischen Feldgeräts (beispielsweise virtuell) ausführen und nachbilden. Der Betriebssicherheitszeitberechnungsalgorithmus ist dazu ausgebildet, auf den Digitalen Zwilling und/oder das Betriebssicherheitsmodell mit den erfassten Sensordaten zuzugreifen, um eine Restbetriebslaufzeit der technischen Komponente zu berechnen.

Der Digitale Zwilling und/oder das Betriebssicherheitsmodell kann ein (beispielsweise Mehrkörpersystem-) Modell umfassen, an das die empfangenen Sensordaten (oder zumindest eine Teilmenge der empfangenen Sensordaten, wobei die Teilmenge festgelegte Arten von Sensordaten umfassen kann) weitergeleitet werden. Die empfangenen Sensordaten können beispielsweise eine Position, Geschwindigkeit, Beschleunigung und/oder ein Patientengewicht umfassen. Alternativ oder ergänzend können die empfangenen Sensordaten eine Kraft, eine momentane Belastung und/oder eine Strahlungsbelastung umfassen. Die, insbesondere mechanische, Kraft und/oder Belastung kann beispielsweise einen Absolutwert und eine Richtung, beispielsweise bezüglich eines Gelenks und/oder Verbindungsstücks der technischen Komponente, umfassen. Alternativ oder ergänzend können die empfangenen Sensordaten die Kraft und/oder Belastung in Einheiten von Newton (N) umfassen. Ferner alternativ oder ergänzend kann die Belastung ein, insbesondere mechanisches, Moment (insbesondere ein Drehmoment, auch: Torsion) umfassen. Die empfangenen Sensordaten können das Moment in Einheiten von Newtonmeter (Nm) umfassen.

Das medizinische Feldgerät kann ein diagnostisches Gerät und/oder ein Behandlungsgerät (z.B. Ultraschall) sein oder umfassen. Das medizinische Feldgerät kann insbesondere ein bildgebendes Gerät, wie beispielsweise ein Ultraschallgerät, ein Magnetresonanztomograph (auch: Kernspintomograph, kurz: MRT), ein Computertomograph (kurz: CT) und/oder ein Röntgengerät, z.B. einen C-Bogen-Gerät, sein oder umfassen. Weiterhin alternativ oder ergänzend kann das medizinische Feldgerät eine Patientenliege (auch: Behandlungsliege) und/oder ein Operationstisch umfassen.

Das medizinische Feldgerät kann mindestens eine technische Komponente (im Folgenden auch kurz: Komponente) umfassen, die einem Verschleiß durch den Betrieb der Komponente/des Feldgerätes unterliegt.

Die technische Komponente kann ein Bauteil des medizinischen Feldgerätes sein. Die technische Komponente kann insbesondere als eine Patientenliege, als ein Stativ (insbesondere ein Deckenstativ oder ein Wandstativ), als ein C-Bogen und/oder als eine Halbleiterbaugruppe ausgebildet sein. Die technische Komponente kann beispielsweise ein Motor, ein Getriebe, eine Spindel, ein Halbleiterbauelement, ein Seilzug und/oder einen Zahnriemen umfassen. Die technische Komponente selbst oder Teile davon sind Gebrauchsgegenstände und unterliegen einem Verschleiß, von dem auch das umfassende medizinische Feldgerät betroffen ist.

Die Gesamtbetriebszeit kann eine Summe über alle Zeiten sein, in denen die technische Komponente und/oder das medizinische Feldgerät in Betrieb gewesen ist, beispielsweise seit einer Erstinstallation und/oder seit einer Wartung.

Die Sensordaten können Bewegungsdaten, beispielsweise eine Geschwindigkeit und/oder Beschleunigung repräsentieren. Alternativ oder ergänzend können die Sensordaten einen Motorstrom und/oder eine Motordrehzahl umfassen. Alternativ oder ergänzend können die Sensordaten eine Kraft und/oder Belastung umfassen, beispielsweise durch ein Gewicht eines Patienten und/oder durch eine mechanische Beanspruchung durch Bedienpersonal des medizinischen Feldgeräts. Weiterhin alternativ oder ergänzend können die Sensordaten eine Strahlenbelastung (beispielsweise in Millisievert, kurz mSv) umfassen.

Eine Art der Sensordaten und/oder des mindestens einen Sensors können von der Art der technischen Komponente abhängig und/oder bestimmt sein.

So werden für eine Patientenliege vorzugsweise Sensordaten von dem Betriebssicherheitszeitberechnungsalgorithmus verwendet, um die Restbetriebszeit zu berechnen, die von folgenden Sensoren erfasst werden:
- Gewichtssensoren und/oder Belastungssensoren, insbesondere zur Erfassung einer Gewichtskraft und/oder einer Belastung, die auf die Patientenliege wirkt (z.B. in Newton, kurz N, und/oder in Newtonmeter, kurz Nm, gemessen), beispielsweise aufgrund des Gewichts eines Patienten und/oder einer Positionsänderung durch Bedienpersonal;
- Optional: Zeitgeber zur Erfassung des Zeitraums, in dem die Gewichtskraft wirkt;
- Optional: Zykluszähler zur Erfassung der Betriebszyklen beim Betrieb der Patientenliege (z.B. Höhenverstellung und/oder Schiebebewegung).

Der Betriebssicherheitszeitberechnungsalgorithmus kann für die Sensordaten der Gewichtssensoren und/oder der Belastungssensoren beispielsweise eine Restlebensdauer berechnen als Differenz einer (beispielsweise vorbestimmten) Gesamtlebensdauer und einer Summe über die jeweiligen gewichtete Sensordaten. Die Gewichtung der Sensordaten kann einen Umrechnungsfaktor der jeweiligen Einheiten der Sensordaten in eine Zeiteinheit umfassen (beispielsweise kann die Gewichtung die Einheit Monate pro Newton für Sensordaten in Newton umfassen). Alternativ oder ergänzend kann die Gewichtung eine Verschleißintensität je nach Größe der jeweiligen empfangenen (insbesondere momentanen und/oder pro Anwendungszyklus) Sensordaten umfassen. Beispielsweise kann eine einmalige Belastung einer Patientenliege mit einer maximalen Gewichtskraft einen höheren Verschleiß bewirken als zwei zeitlich aufeinander folgende Belastungen der Patientenliege mit jeweils der Hälfte der maximalen Gewichtskraft. Alternativ oder ergänzend kann eine Restlebensdauer einer einmalig mit dem maximalen Gewicht belasteten Patientenliege kürzer sein als eine Restlebensdauer der zweimalig mit der Hälfte des maximalen Gewichts belasteten Patientenliege.

So werden für ein Deckenstativ, das zumindest in einer vertikalen Achse (Z-Achse) beweglich ist vorzugsweise Sensordaten von dem Betriebssicherheitszeitberechnungsalgorithmus verwendet, um die Restbetriebszeit zu berechnen, die von folgenden Sensoren erfasst werden:
- Gewichtssensoren insbesondere zur Erfassung einer Gewichtskraft, die auf das Deckenstativ wirken;
- Optional: Zykluszähler, der die Betriebszyklen zählt, wie oft, das Deckenstativ translatorisch nach oben oder unten bewegt worden ist;
- Optional und falls das Deckenstativ auch in einer horizontalen Ebene bewegbar ist (X- und /oder Y-Achse): einen Zykluszähler und/oder Kraftsensor zur Messung der Betriebszyklen, in denen das Deckenstativ in der X. und/oder Y-Achse bewegt worden ist und mit welcher Kraft;
- Optional: Zeitgeber zur Erfassung des Zeitraums, in dem die Gewichtskraft wirkt;
- Optional: Insbesondere kann sensorisch mittels Kraftsensoren erfasst werden, welche Kraft auf das Stativ wirkt, wenn es sich in einer seiner maximalen Bewegungsposition befindet (also z.B. ganz oben oder ganz unten)

Beispielsweise kann für ein Deckenstativ mit einer Seilzugmechanik (oder kurz: Seilzug) ein Austausch des Seilzugs nach einer vorkonfigurierbaren Anzahl von Höhenverstellbewegungen im Betriebssicherheitszeitberechnungsalgorithmus vorgesehen sein. Die vorkonfigurierbare Anzahl kann in einem Bereich zwischen 40.000 und 60.000 Höhenverstellungen und insbesondere bei 50.000 Höhenverstellungen in Summe liegen.

Für ein Wandstativ (das an einer vertikalen Stützkonstruktion, z.B. einer Wand angeordnet ist) und vertikal beweglich ist können die im Zusammenhang mit dem Deckenstativ genannten Sensoren verwendet werden.

Beispielsweise kann der Sensor für eine technische Komponente in der Ausbildung als Halbleiterbauelement ein Dosimeter sein, und die Sensordaten können eine emittierte/empfangene Strahlenbelastung codieren (gemessen in Milli Sievert, mSv). Beispielsweise kann für eine Leiterplatte in einer Tiefenblende als Halbleiterbauelement gemäß dem Betriebssicherheitszeitberechnungsalgorithmus ein Austausch nach einer konfigurierbaren Wert einer kumulierten Strahlendosis (und/oder Gesamtstrahlendosis über eine Gesamtbetriebszeit) vorgesehen sein. Die kumulierte Strahlendosis kann in einem Bereich zwischen 5Sv und 15Sv und insbesondere bei 10Sv konfiguriert werden.

Der Sensor kann alternativ oder kumulativ ein Temperatursensor, ein Zykluszähler (z.B. für ausgeführte Betriebszyklen von Aktoren), ein Kraftsensor, ein Drehmomentsensor, ein Beschleunigungssensor, ein Positionssensor, ein Bewegungssensor und/oder ein Sensor zur Erfassung von Parametern sein, die indikativ für einen Verschleißzustand der Komponente sind.

Der Schritt des Empfangens kann ein Umwandeln der Sensordaten in ein vorbestimmtes Datenformat umfassen.

Das Datenformat kann auch als Datenart und/oder Datentyp bezeichnet werden.

Der Schritt des Speicherns kann ein Speichern der Sensordaten in dem vorbestimmten Datenformat umfassen. Alternativ oder ergänzend kann der Schritt des Speicherns ein Bestimmen eines Gesamtwerts der empfangenen Sensordaten (beispielsweise seit einer Installation und/oder seit einer Wartung der technischen Komponente) von dem mindestens einen Sensor umfassen. Beispielsweise kann mittels des Dosimeters eine Gesamtstrahlenbelastung bestimmt und im Schritt des Speichers gespeichert werden.

Der Datenspeicher kann, beispielsweise lokal, an dem medizinischen Feldgerät angeordnet sein. Alternativ oder ergänzend kann der Datenspeicher an einem medizinischen Standort, beispielsweise an einem Krankenhaus und/oder einer Diagnosepraxis (beispielsweise einer Röntgenpraxis) angeordnet sein. Die Anordnung an einem medizinischen Feldgerät und/oder einem medizinischen Standort kann als dezentral bezeichnet werden. Weiterhin alternativ oder ergänzend kann das medizinische Feldgerät und/oder der medizinische Standort mit einem zentralen Datenspeicher und/oder einer zentralen Verarbeitungseinheit verbunden sein. Beispielsweise kann ein zentraler Datenspeicher und/oder eine zentrale Verarbeitungsarbeit über drahtgebundene (auch: kabelgebundene) und/oder drahtlose Datenverbindungen mit mehreren medizinischen Feldgeräten, insbesondere an mehreren medizinischen Standorten, verbunden sein.

Der Gesamtwert der empfangenen Sensordaten eines Sensors kann eine Summe über die Sensordaten eines Sensors während der Gesamtbetriebszeit umfassen, beispielsweise eine Gesamtstrahlenbelastung (beispielsweise die Summe über alle Strahlenbelastungen in Einheiten von mSv). Alternativ oder ergänzend kann der Gesamtwert ein Histogramm und/oder eine Klassifizierung der Sensordaten nach Klassen umfassen. Eine Klasse (englisch auch: "Bin") kann beispielsweise einen Bereich eines Gewichts, einer Geschwindigkeit und/oder einer Beschleunigung umfassen.

Alternativ oder ergänzend kann ein Betriebssicherheitsmodell mindestens einen Referenzwert je nach Art der Sensordaten umfassen. Der Referenzwert kann einen Maximalwert umfassen, beispielsweise einer einmaligen Belastung und/oder einer Summe von Belastungen. Alternativ oder ergänzend kann ein Digitaler Zwilling eine virtuelle Kopie der technischen Komponente umfassen. Mittels des Digitalen Zwillings kann eine momentane und/oder über eine Betriebszeit der technischen Komponente bestimmte Belastung anhand der empfangenen und gespeicherten Sensordaten bestimmt werden.

Der Schritt des Zugreifens, um eine Restbetriebslaufzeit zu berechnen, kann einen Vergleich des Gesamtwerts einer Art von Sensordaten mit einem Referenzwert, beispielsweise dem Maximalwert, umfassen.

Der mindestens eine Referenzwert kann einen Schwellenwert (auch: Toleranzschwelle) umfassen. Beispielsweise kann ein Gesamtwert, der den Schwellenwert (auch: Toleranzschwelle) überschreitet, einen Verschleiß der technischen Komponente und/oder des medizinischen Feldgeräts indizieren.

Der Schritt des Ausgebens der berechneten Restbetriebslaufzeit als Zwischenergebnis kann lokal, beispielsweise an einer Ausgabeeinheit des medizinischen Feldgeräts, und/oder dezentral, beispielsweise an einer Ausgabeeinheit des medizinischen Standorts, ausgeführt werden. Alternativ oder ergänzend kann der Schritt des Ausgebens ein Ausgeben an einer zentralen Ausgebeeinheit umfassen. Beispielsweise kann einem zentralen Datenspeicher und/oder einer zentralen Verarbeitungseinheit eine zentrale Ausgabeeinheit zugeordnet sein.

Das mindestens eine Steuersignal kann ein Abschalten des medizinischen Feldgeräts umfassen. Alternativ oder ergänzend kann das mindestens eine Steuersignal einen Funktionsumfang und/oder mindestens einen Funktionsparameter (auch: Betriebsparameter) für den Betrieb des Feldgeräts einschränken. Der Funktionsumfang und/oder der mindestens eine Funktionsparameter können der Steuergröße entsprechen und/oder die Steuergröße umfassen. Weiterhin alternativ oder ergänzend kann mindestens ein Steuersignal an einen Wartungsdienst und/oder eine externe Serviceeinheit gesendet werden.

Das Sperren und/oder Abschalten des medizinischen Feldgeräts kann eine mechanische Sperre, eine elektrische und/oder eine elektronische Sperre verwenden.

Das Einschränken des Betriebs des medizinischen Feldgeräts (mittels des Steuersignals) kann:
- eine Reduzierung einer ausführbaren Geschwindigkeit und/oder Beschleunigung, eine Beschränkung einer Höhenverstellbarkeit im Falle eines Decken- oder Wandstativs oder eines C-Bogens sein; So kann das Steuersignal z.B. die Bewegbarkeit des Stativs einschränken, so dass dieses nur noch im oberen oder unteren Bereich betrieben werden kann und/oder, dass es nicht mehr in X-/Y-Richtung verstellbar ist und nur noch in Z-Richtung; kumulativ oder ergänzend kann das Steuersignal bewirken, dass die technische Komponente (Z.B. das Stativ oder der C-Bogen) nur noch langsam oder in einem eingeschränkten Bewegungsbereich (z.B. bei einem C-Bogen: in einem eingeschränkten Winkelbereich) bewegt werden kann; dies hat den technischen Hintergrund, dass z.B. ein Bauteil, wie ein Seilzug bei einem Deckenstativ vorzeitig altern kann und/oder
- eine Begrenzung einer Strahlenintensität und/oder einer Strahlendosis im Fall einer Halbleiterbaugruppe sein. Dies hat den technischen Hintergrund, dass die elektronischen Komponenten durch eine Strahlenbelastung vorzeitig altern oder sogar ausfallen;
- eine Beschränkung des Bewegungsraums oder des zulässigen Maximalgewichts im Falle der Ausbildung der technischen Komponente als Patientenliege; So kann z.B. das Steuersignal bewirken, dass die Patientenliege nur noch für Patienten mit einem Gewicht unter einem konfigurierbaren festgelegten Wert (beispielsweise 100 Kilogramm) betrieben werden kann oder nur noch in einem eingeschränkten Z-Achsen Bereich höhenverstellt werden kann.

Kumulativ oder alternativ kann das mindestens eine Steuersignal eine Verschleißindikation der technischen Komponente, und/oder mindestens eines Bauelements der technischen Komponente, des medizinischen Feldgerätes umfassen.

Durch das (beispielsweise wahlweise) auf Basis der berechneten Betriebslaufzeit Ausgeben mindestens eines Steuersignals kann eine Betriebssicherheit des medizinischen Feldgeräts verbessert werden. Alternativ oder ergänzend kann durch das wahlweise Ausgeben mindestens eines Steuersignals ein (beispielsweise gezielter) Austausch der technischen Komponente, und/oder mindestens eines Bauelements der technischen Komponente, eingeleitet werden. Durch den (beispielsweise gezielten) Austausch der technischen Komponente, und/oder des mindestens einen Bauelements der technischen Komponente, kann eine Gesamtbetriebsdauer des medizinischen Feldgeräts verlängert werden.

Das Verfahren kann ferner einen Verfahrensschritt des Verarbeitens der Sensordaten umfassen. Das Verarbeiten kann ein Umwandeln der Sensordaten in ein vorbestimmtes Datenformat umfassen. Die Patientenliege kann zu Diagnosezwecken und/oder Behandlungszwecken höhenverstellbar und in mindestens eine horizontale Richtung bewegbar sein. Beispielsweise kann die Patientenliege einen Behandlungstisch und/oder Operationstisch umfassen, oder in einem Behandlungstisch und/oder Operationstisch umfasst sein, und der Behandlungstisch und/oder Operationstisch kann höhenverstellbar sein. Alternativ oder ergänzend kann die Patientenliege einem bildgebenden System zugeordnet sein, beispielsweise einem Magnetresonanztomographen (MRT), einem Ultraschallgerät, einem Computertomographen (CT) und/oder einem Röntgenapparat.

Das Deckenstativ kann auch als Strahlenstativ bezeichnet werden. Alternativ oder ergänzend kann das Deckenstativ einen an einer Zimmerdecke montierten Hebelarm umfassen, an dessen der Zimmerdecke abgewandten Ende ein bildgebendes System, beispielsweise ein Röntgenapparat angeordnet ist. Mittels des Deckenstativs kann das bildgebende System höhenverstellbar sein. Alternativ oder ergänzend kann das Deckenstativ einen Seilzug umfassen zur Höhenverstellung des der Zimmerdecke abgewandten Endes des Deckenstativs. Ein Verschleiß des Deckenstativs kann durch einen Verschleiß des Seilzugs verursacht oder mitverursacht sein.

Alternativ oder ergänzend kann das Stativ mindestens eine Schiene umfassen. Beispielsweise kann die Schiene zwischen einem Boden und einer Decke eines Behandlungszimmers und/oder eines Untersuchungszimmers montiert sein. An der Schiene kann beispielsweise ein Röntgengerät vertikal verfahrbar sein. Alternativ oder ergänzend kann die mindestens eine Schiene an der Decke des Behandlungszimmers und/oder des Untersuchungszimmers montiert sein, und das Deckenstativ kann in mindestens eine Richtung horizontal verfahrbar sein.

Der C-Bogen kann eine Halterung für ein bildgebendes System umfassen, beispielsweise einen Röntgenstrahler und/oder einen Bildempfänger. Alternativ oder ergänzend kann der C-Bogen einen oder mehrere translatorische Freiheitsgrade, beispielsweise horizontal und/oder vertikal, und/oder einen oder mehrere rotatorische Freiheitsgrade, beispielsweise eine orbitale Rotation und/oder eine Schwenkung, umfassen. Alternativ oder ergänzend kann der C-Bogen einen Zahnriemen umfassen. Ein Verschleiß des C-Bogens kann durch einen Verschleiß des Zahnriemens verursacht oder mitverursacht werden.

Die Halbleiterbaugruppe kann zu einer Steuerung und/oder einer Auswertung des medizinischen Feldgeräts ausgebildet sein. Ein Verschleiß der Halbleiterbaugruppe kann durch eine chemische Zustandsänderung von Elementen der Halbleitergruppe verursacht oder mitverursacht sein, beispielsweise aufgrund von Strahlung. Die Sensordaten können mindestens eine Art von Sensordaten umfassen, die ausgewählt ist aus der Gruppe bestehend aus einer Position, einem Gewicht, mit dem die technische Komponente belastet wird, einer Geschwindigkeit, einer Beschleunigung, einer Temperatur, einer Strahlendosis, einer Betriebsdauer, und einer Anzahl Benutzungszyklen.

Die Position kann eine Höhe und/oder eine Orientierung im Raum der technischen Komponente und/oder des medizinischen Feldgeräts umfassen.

Das Gewicht kann ein Patientengewicht und/oder ein Gewicht von Medizinprodukten umfassen, mit denen die technische Komponente und/oder das medizinische Feldgerät belastet wird.

Die Geschwindigkeit kann sich auf eine Komponente beziehen und eine Geschwindigkeit einer Positionsänderung, beispielsweise der Patientenliege und/oder einer Zustandsänderung, beispielsweise eine Winkeländerung zwischen zwei Armstücken des Hebelarms des Deckenstativs, umfassen.

Die Temperatur kann eine Zimmertemperatur, eine Temperatur während eines Diagnosevorgangs, beispielsweise einer Benutzung eines bildgebenden Systems, und/oder während einer Operation umfassen. Alternativ oder ergänzend kann die Temperatur eine Körpertemperatur eines Patienten, beispielsweise auf der Patientenliege umfassen. Weiterhin alternativ oder ergänzend kann die Temperatur eine Betriebstemperatur der Halbleiterbaugruppe umfassen.

Die Strahlendosis kann kumulativ erfasst werden, beispielsweise als Gesamtstrahlendosis seit erster Inbetriebnahme der technischen Komponente, insbesondere einer Halbleiterbaugruppe, und/oder des medizinischen Feldgeräts. Alternativ oder ergänzend kann die Strahlendosis mittels eines, beispielsweise digitalen, Dosimeters bestimmt werden.

Die Betriebsdauer kann eine Zeitspanne umfassen, während der die technische Komponente und/oder das medizinische Feldgerät (beispielsweise ununterbrochen) aktiviert und/oder in Benutzung ist. Alternativ oder ergänzend kann die Betriebsdauer einer oder mehreren Arten von Sensordaten zugeordnet sein. Beispielsweise kann die Betriebsdauer in Kombination mit einem (z.B. jeweiligen) Gewicht eines Patienten auf einer Patientenliege bestimmt werden.

Die Anzahl von Benutzungszyklen kann durch eine Anzahl von Benutzungen definiert sein. Beispielsweise kann die Anzahl Benutzungszyklen einer Patientenliege einer Anzahl von MRT-Scans, einer Anzahl von Fahrten in eine Röhre eines bildgebenden Systems und/oder einer Anzahl von Einsätzen eines Deckenstativs, beispielsweise umfassend ein Röntgengerät, entsprechen.

Das Erzeugen mindestens eines Steuersignals kann ferner ein Versenden einer Verschleißindikation an eine Steuerung, an eine externe Serviceeinheit und/oder an den Datenspeicher umfassen. Das Versenden des Verschleißzustandes bzw. der Verschleißindikation der technischen Komponente und/oder des medizinischen Feldgeräts kann eine Anzeige an dem medizinischen Feldgerät umfassen, beispielsweise auf einem Bildschirm und/oder mittels einer Kontrollleuchte.

Das Versenden der Verschleißindikation kann ferner eine Datenübertragung an eine externe Datenverarbeitungsanlage, insbesondere umfassend einen zentralen Datenspeicher, umfassen. Die Datenübertragung kann eine drahtlose und/oder kabelgebundene (auch: drahtgebundene) Datenübertragung umfassen.

Der Datenspeicher und/oder die Verarbeitungseinheit kann an dem medizinischen Feldgerät, an einem medizinischen Standort umfassend das medizinische Feldgerät und/oder an einem zentralen Standort, der zum Datenaustausch mit einem oder mehreren medizinischen Standorten ausgebildet ist, angeordnet sein.

In Reaktion auf das Ausgeben des mindestens einen Steuersignals kann eine manuelle und/oder automatische Änderung, insbesondere der mindestens einen Steuergröße, ermöglicht werden und/oder erfolgen.

Die manuelle und/oder automatische Änderung kann als ein Zurücksetzen der Einstellungen der technischen Komponente und/oder des medizinischen Feldgeräts ausgebildet sein. Alternativ oder ergänzend kann die manuelle und/oder automatische Änderung als eine Änderung von Betriebsparametern (auch: Funktionsparametern), beispielsweise zu vorbestimmten Werten für einen Regelbetrieb, ausgebildet sein.

In der Verarbeitungseinheit kann mindestens ein Referenzwert für die Sensordaten eines Sensors abgelegt sein. Alternativ oder ergänzend kann der Digitale Zwilling und/oder das Betriebssicherheitsmodell mindestens einen Referenzwert für die Restbetriebslaufzeit in Abhängigkeit von den Sensordaten eines Sensors beinhalten.

Das Betriebssicherheitsmodell kann einen Referenzwert je Klasse der empfangenen Sensordaten eines Sensors beinhalten, beispielsweise je Intervall eines Gewichts eines Patienten. Alternativ oder ergänzend kann der mindestens eine Referenzwert eine Zuordnung einer Anzahl von Benutzungszyklen (auch: Betriebszyklen und/oder Bewegungszyklen) beinhalten. Beispielsweise kann hundert (100) Benutzungszyklen eine vorbestimmte Restbetriebslaufzeit in Einheiten von Jahren, Monaten und/oder Tagen zugeordnet sein.

Alternativ oder ergänzend kann der mindestens eine Referenzwert einen Durchschnittswert, einen Erfahrungswert und/oder einen, insbesondere mittels des Digitalen Zwillings und/oder des Betriebssicherheitsmodells, bestimmten Wert für eine Restbetriebslaufzeit beinhalten.

Weiterhin alternativ oder ergänzend kann der mindestens eine Referenzwert einen Vergleich des Durchschnittswerts und/oder des Erfahrungswerts mit dem, insbesondere mittels des Digitalen Zwillings und/oder des Betriebssicherheitsmodells, bestimmten Wert umfassen. Beispielsweise kann eine Fehlermeldung ausgegeben und/oder (z.B. mittels automatischer und/oder manueller Eingabe) technische Diagnosemaßnahmen eingeleitet werden, wenn der Vergleich eine Abweichung über eine vorbestimmte Toleranzschwelle (auch: Schwellenwert) hinaus ergibt.

Vorstehend wurde die Lösung der Aufgabe anhand des Verfahrens beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die gegenständlichen Ansprüche (die beispielsweise auf eine Vorrichtung, ein System oder auf ein Computerprogrammprodukt gerichtet sind) mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Gemäß einem zweiten Aspekt wird die Aufgabe durch eine Vorrichtung zur Bestimmung und Durchsetzung einer Betriebssicherheit einer technischen Komponente eines medizinischen Feldgeräts in Abhängigkeit von über eine Gesamtbetriebszeit der technischen Komponente mittels mindestens eines Sensors überwachten Sensordaten des medizinischen Feldgeräts gelöst. Die Vorrichtung umfasst mindestens eine Recheneinheit. Die Recheneinheit umfasst eine Empfangsschnittstelle, die ausgebildet ist zum Empfangen von Sensordaten mittels des mindestens einen Sensors während einer Betriebszeit der technischen Komponente des medizinischen Feldgeräts. Die Recheneinheit umfasst ferner einen Speicher bzw. Datenspeicher, der ausgebildet ist zum Speichern der mittels des mindestens einen Sensors empfangenen Sensordaten. Die Recheneinheit ist ferner ausgebildet zum Zugreifen auf eine Verarbeitungseinheit, in der ein Digitaler Zwilling und/oder ein Betriebssicherheitsmodell gespeichert ist. Das Zugreifen erfolgt mit den empfangenen Sensordaten, um auf Basis eines in der Verarbeitungseinheit gespeicherten Betriebssicherheitszeitberechnungsalgorithmus eine Restbetriebslaufzeit der technischen Komponente zu berechnen. Die Recheneinheit umfasst ferner eine Ausgabeeinheit, die ausgebildet ist zum Ausgeben der berechneten Restbetriebslaufzeit der technischen Komponente als Zwischenergebnis. Die Recheneinheit ist ferner ausgebildet zum Erzeugen mindestens eines Steuersignals zum Steuern des medizinischen Feldgerätes auf Basis der berechneten Restbetriebslaufzeit, um den Betrieb des medizinischen Feldgerätes einzuschränken, vollständig zu sperren oder mit mindestens einer veränderten Steuergröße fortzusetzen.

Die Vorrichtung kann ferner dazu ausgebildet sein, die oben im Zusammenhang mit dem Verfahren beschriebenen Merkmale auszuführen oder zu implementieren.

In einer vorteilhaften Ausführungsform der Erfindung kann das Verfahren lokal auf oder an der technischen Komponente ausgeführt werden, also entweder direkt auf der Komponente selbst oder auf einem diesem zugeordneten Gerät (z.B. Steuergerät). Insbesondere kann die Vorrichtung eine Umwandlungseinheit umfassen, die ausgebildet ist, zum Umwandeln der Sensordaten in ein vorbestimmtes Datenformat.

Die technische Komponente des medizinischen Feldgerätes kann sein:
- eine Patientenliege;
- ein Stativ, insbesondere ein Deckenstativ;
- ein C-Bogen; und/oder
- eine Halbleiterbaugruppe.

Die Sensoren können unterschiedlicher Art sein, wie z.B. ein Positionssensor, ein Gewichtssensor (Waage), ein Geschwindigkeitssensor, ein Gyrosensor oder ein Beschleunigungssensor, ein Temperaturfühler, ein Dosimeter, und/oder ein Zykluszähler, der zur Messung der Anzahl der Betriebszyklen bestimmt ist.

In einer weiteren vorteilhaften Ausführungsform der Erfindung kann das Verfahren auch verteilt ausgeführt werden. Ebenso ist es möglich, das Verfahren auf einem zentralen Server, z.B. cloudbasiert auszuführen. Dies hat den Vorteil, dass das Verfahren als Service angeboten werden kann für eine Menge von Krankenhäusern mit mehreren Feldgeräten und/oder technischen Komponenten.

Gemäß einem dritten Aspekt wird die Aufgabe somit durch ein System zur Bestimmung und Durchsetzung einer Betriebssicherheit einer technischen Komponente eines medizinischen Feldgeräts in Abhängigkeit von über eine Gesamtbetriebszeit der technischen Komponente mittels mindestens eines Sensors überwachten Sensordaten des medizinischen Feldgeräts gelöst. Das System umfasst mindestens eine Vorrichtung und/oder mindestens eine lokale Recheneinheit, die dazu ausgebildet ist, das Verfahren gemäß dem ersten Aspekt auszuführen. Die mindestens eine oder jede Vorrichtung und/oder lokale Recheneinheit umfasst mindestens eine Datenschnittstelle. Das System umfasst ferner mindestens einen Sensor zur Erfassung der Sensordaten. Der mindestens eine Sensor umfasst eine Datenschnittstelle zum Senden der Sensordaten an mindestens eine Vorrichtung und/oder lokale Recheneinheit. Das System umfasst ferner einen Server, der einen Datenspeicher, eine Verarbeitungseinheit und eine Datenschnittstelle umfasst. Die Datenschnittstelle des Servers ist zum Senden und Empfangen von Daten an die mindestens eine Vorrichtung und/oder lokale Recheneinheit ausgebildet. Der Datenspeicher des Servers ist zum Speichern der vom mindestens einen Sensor über die mindestens eine Vorrichtung und/oder lokale Recheneinheit empfangenen Sensordaten ausgebildet. In der Verarbeitungseinheit des Servers ist ein Digitaler Zwilling und/oder ein Betriebssicherheitsmodell gespeichert, um auf Basis eines in der Verarbeitungseinheit gespeicherten Betriebssicherheitszeitberechnungsalgorithmus eine Restbetriebslaufzeit der technischen Komponente zu berechnen.

Der Server kann einer Vielzahl von medizinischen Feldgeräten und/oder technischen Komponenten zugeordnet sein. Alternativ oder ergänzend kann der Server einer Mehrzahl von medizinischen Standorten zugeordnet sein. Jeder medizinische Standort kann mindestens ein medizinisches Feldgerät mit mindestens einer technischen Komponente umfassen.

Das System des dritten Aspekts kann ferner eines oder mehrere der Merkmale des ersten Aspekts umfassen.

Eine weitere Aufgabenlösung besteht in einem Computerprogrammprodukt, das in einen Speicher eines Computers geladen oder ladbar ist mit Computerprogrammcode zur Durchführung des oben näher beschriebenen Verfahrens, wenn das Computerprogrammprodukt auf dem Computer ausgeführt wird.

Eine weitere Aufgabenlösung sieht ein Computerprogramm vor, mit Computerprogrammcode zur Durchführung aller Verfahrensschritte des oben näher beschriebenen Verfahrens, wenn das Computerprogramm auf einem Computer ausgeführt wird. Dabei ist es auch möglich, dass das Computerprogramm auf einem von einem Computer lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Fig. 1: ein Ablaufdiagramm eines Verfahrens gemäß einer bevorzugten Ausführungsform der Erfindung;
- Fig. 2: ein Blockschaltbild einer Vorrichtung gemäß einer bevorzugten Ausführungsform der Erfindung;
- Fig. 3: ein erstes Ausführungsbeispiel einer technischen Komponente eines medizinischen Feldgeräts;
- Fig. 4: drei weitere Ausführungsbeispiele technischer Komponenten medizinischer Feldgeräte;
- Fig. 5: ein Ausführungsbeispiel eines Datenaustausches zwischen einer Mehrzahl technischer Komponenten von medizinischen Feldgeräten, denen jeweils eine Vorrichtung gemäß Fig. 2 zugeordnet sein kann, mit einem Server; und
- Fig. 6: ein Ausführungsbeispiel eines Betriebssicherheitszeitberechnungsalgorithmus.

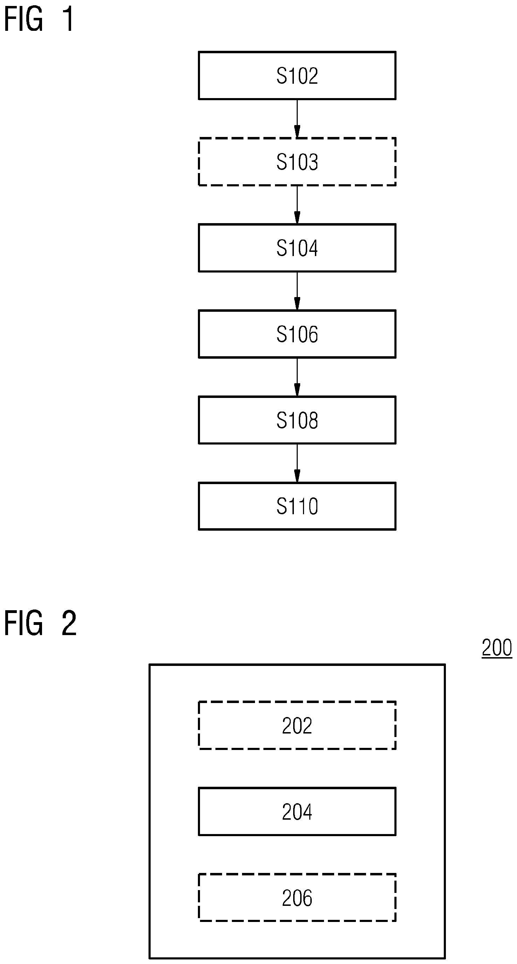

Fig. 1 zeigt ein erfindungsgemäßes Verfahren 100 zur Bestimmung und Durchsetzung einer Betriebssicherheit einer technischen Komponente eines medizinischen Feldgeräts in Abhängigkeit von über eine Gesamtbetriebszeit der technischen Komponente mittels mindestens eines Sensors überwachten Sensordaten des medizinischen Feldgeräts. Das Verfahren 100 umfasst einen Schritt S102 des Empfangens von Sensordaten mittels des mindestens einen Sensors während einer Betriebszeit der technischen Komponente des medizinischen Feldgeräts. Das Verfahren 100 umfasst ferner einen Schritt S104 des Speicherns der mittels des mindestens einen Sensors empfangenen Sensordaten in einem Datenspeicher. Das Verfahren 100 umfasst ferner einen Schritt S106 des Zugreifens auf eine Verarbeitungseinheit, in der ein Digitaler Zwilling und/oder ein Betriebssicherheitsmodell gespeichert ist. Das Zugreifen S106 mit den empfangenen Sensordaten erfolgt, um auf Basis eines in der Verarbeitungseinheit gespeicherten Betriebssicherheitszeitberechnungsalgorithmus eine Restbetriebslaufzeit der technischen Komponente zu berechnen.

Das Verfahren 100 umfasst ferner einen Schritt S108 des Ausgebens der berechneten Restbetriebslaufzeit der technischen Komponente als Zwischenergebnis. Das Verfahren 100 umfasst ferner einen Schritt S110 des Erzeugens mindestens eines Steuersignals zum Steuern des medizinischen Feldgerätes auf Basis der berechneten Restbetriebslaufzeit, um den Betrieb des medizinischen Feldgerätes (beispielsweise wahlweise) einzuschränken, vollständig zu sperren oder mit mindestens einer veränderten Steuergröße fortzusetzen.

Optional umfasst das Verfahren 100 einen Schritt S103 des Verarbeitens der Sensordaten. Das Verarbeiten S103 kann ein Umwandeln der Sensordaten in ein vorbestimmtes Datenformat umfassen.

Das Verfahren kann mittels mindestens einer Vorrichtung ausgeführt werden. Alternativ oder ergänzend kann das Verfahren mittels eines Systems umfassend mindestens eine Vorrichtung und/oder lokale Recheneinheit und einen (insbesondere externen) Server ausgeführt werden.

Fig. 2 zeigt eine erfindungsgemäße Vorrichtung 200 zur Bestimmung und Durchsetzung einer Betriebssicherheit einer technischen Komponente eines medizinischen Feldgeräts in Abhängigkeit von über eine Gesamtbetriebszeit der technischen Komponente mittels mindestens eines Sensors überwachten Sensordaten des medizinischen Feldgeräts. Die Vorrichtung 200 umfasst mindestens eine Recheneinheit 204, die ausgebildet ist zum Empfangen von Sensordaten mittels des mindestens einen Sensors während einer Betriebszeit der technischen Komponente des medizinischen Feldgeräts, beispielsweise mittels der Empfangsschnittstelle oder der optionalen Datenschnittstelle 202. Die Recheneinheit 204 ist ferner ausgebildet zum Speichern der mittels des mindestens einen Sensors empfangenen Sensordaten in einem Datenspeicher, beispielsweise einem optionalen lokalen Datenspeicher 206. Die Recheneinheit 204 ist ferner ausgebildet zum Zugreifen auf eine Verarbeitungseinheit, in der ein Digitaler Zwilling und/oder ein Betriebssicherheitsmodell gespeichert ist. Das Zugreifen erfolgt mit den empfangenen Sensordaten, um auf Basis eines in der Verarbeitungseinheit gespeicherten Betriebssicherheitszeitberechnungsalgorithmus eine Restbetriebslaufzeit der technischen Komponente zu berechnen.

Die Recheneinheit 204 umfasst ferner eine Ausgabeeinheit, die zum Ausgeben der berechneten Restbetriebslaufzeit der technischen Komponente als Zwischenergebnis ausgebildet ist.

Die Recheneinheit 204 ist ferner ausgebildet zum Erzeugen mindestens eines Steuersignals zum Steuern des medizinischen Feldgerätes auf Basis der berechneten Restbetriebslaufzeit, um den Betrieb des medizinischen Feldgerätes (beispielsweise wahlweise) einzuschränken, vollständig zu sperren oder mit mindestens einer veränderten Steuergröße fortzusetzen.

Die optionale Datenschnittstelle 202 kann ferner dazu ausgebildet sein, mit einem (insbesondere externen) Server in Datenaustausch zu stehen.

Der Datenspeicher kann außerhalb der Vorrichtung 200 und/oder räumlich getrennt von der Recheneinheit 204 angeordnet sein. Beispielsweise kann der Datenspeicher an dem (insbesondere externen) Server angeordnet sein. Alternativ oder ergänzend kann der Datenspeicher einen lokalen Datenspeicher 206 der Vorrichtung 200 umfassen.

Die Vorrichtung 200 kann dazu ausgebildet sein, das Verfahren 100 auszuführen.

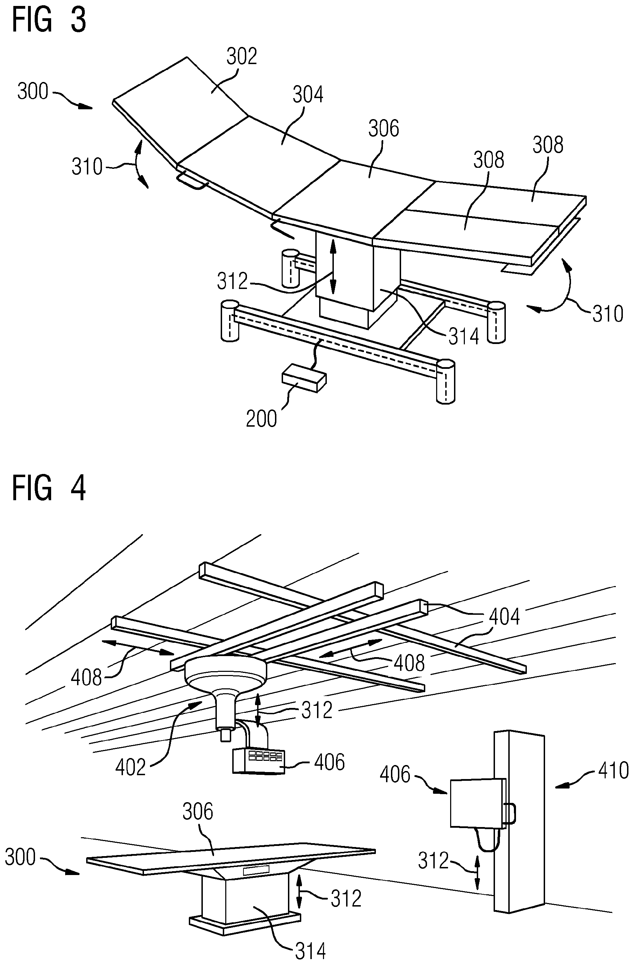

Fig. 3 zeigt ein erstes Ausführungsbeispiel einer technischen Komponente eines medizinischen Feldgeräts als Patientenliege 300. Die Patientenliege umfasst beispielhaft ein verstellbares Kopfende 302, eine verstellbare Rumpfauflagefläche 304, eine horizontale Auflagefläche 306 und eine oder zwei verstellbare Beinauflageflächen 308. Das Kopfende kann beispielsweise mittels einer Rotationsbewegung 310 verstellt werden. Die Auflagefläche 306 kann höhenverstellbar 312 sein mittels eines Bodenstativs 314, das höhenverstellbar 312 ist. Optional kann das Bodenstativ 314 ferner horizontal verfahrbar sein (nicht gezeigt).

Das Fußende kann mittels einer Rotationsbewegung 310, beispielsweise separat pro Beinauflagefläche 308, verstellbar sein. Ein oder mehrere der Verstellvorgänge können von der Vorrichtung 200 erfasst und/oder gesteuert werden.

Fig. 4 zeigt drei weitere Ausführungsbeispiele technischer Komponenten medizinischer Feldgeräte.

Eine Patientenliege 300 in Fig. 4 umfasst eine horizontale Auflagefläche 306, die mittels eines Bodenstativs 314 höhenverstellbar 312 ist. Die Patientenliege 300 kann als einfachere Ausführung der Patientenliege 300 verstanden werden, die oben in Zusammenhang mit Fig. 3 beschrieben worden ist. Alternativ kann die Auflagefläche 306 horizontal verfahrbar sein (nicht gezeigt), beispielsweise entlang einer Längsrichtung zum Einführen in eine Röhre eines bildgebenden Systems.

Das Bodenstativ 314 kann ein oder mehrere Gelenkstücke (nicht gezeigt) umfassen zur Höhenverstellung 312 und/oder horizontalen Verstellung (nicht gezeigt). Die Restbetriebslaufzeit der Patientenliege kann durch einen Verschleiß der Gelenkstücke begrenzt sein.

Ein verstellbares Deckenstativ 402 - als weiteres Ausführungsbeispiel in Fig. 4 - ist höhenverstellbar 312 und/oder horizontal verstellbar 408. Die horizontale Verstellung 408 kann beispielsweise mittels zwei zueinander senkrecht angeordneter Schienensysteme 404 erfolgen, die jeweils einer von zwei zueinander orthogonalen, horizontal angeordneten Verstellrichtungen 408 zugeordnet sind. Das verstellbare Deckenstativ 402 kann ein Röntgengerät 406 umfassen.

Mittels der Verstellungen 312; 408 des Deckenstativs 402 kann das Röntgengerät 406 beispielsweise bezüglich eines auf der Patientenliege 300 positionierten Patienten angeordnet werden, um ein Röntgenbild (beispielsweise eines Teils des Körpers des Patienten, in einem vorbestimmten Abstand, in einer vorbestimmten Schärfe und/oder in einer vorbestimmten Auflösung) aufzunehmen.

In einem weiteren (nicht gezeigten) Ausführungsbeispiel kann die Patientenliege 300 horizontal verfahrbar sein, beispielsweise mittels am Boden angeordneten Schienen. Die horizontal verfahrbare Patientenliege 300 kann beispielsweise in ein CT oder MRT eingeführt werden.

Ein verstellbares Wandstativ 410 - als weiteres Ausführungsbeispiel in Fig. 4 - umfasst ein höhenverstellbares 312 Röntgengerät 406. Das Wandstativ 410 kann fest an einer Wand eines Behandlungszimmers und/oder eines Untersuchungszimmers montiert sein.

Im Folgenden wird das Verfahren anhand eines beispielhaften Systems beschrieben.

Die Schritte des Empfangens S102 und Speicherns S104 der Sensordaten, des Berechnens S106 und Ausgebens S108 einer Restbetriebslaufzeit (auch kurz: Restbetriebszeit) anhand eines Betriebssicherheitsmodells sowie das Erzeugen S110 entsprechender Steuersignale können auch als "Realtime Tracking" und Aktualisierung der Restbetriebszeit von medizinischen Feldgeräten (und/oder technischen Komponenten medizinischer Feldgeräte) und/oder medizinischen Systemen bezeichnet werden.

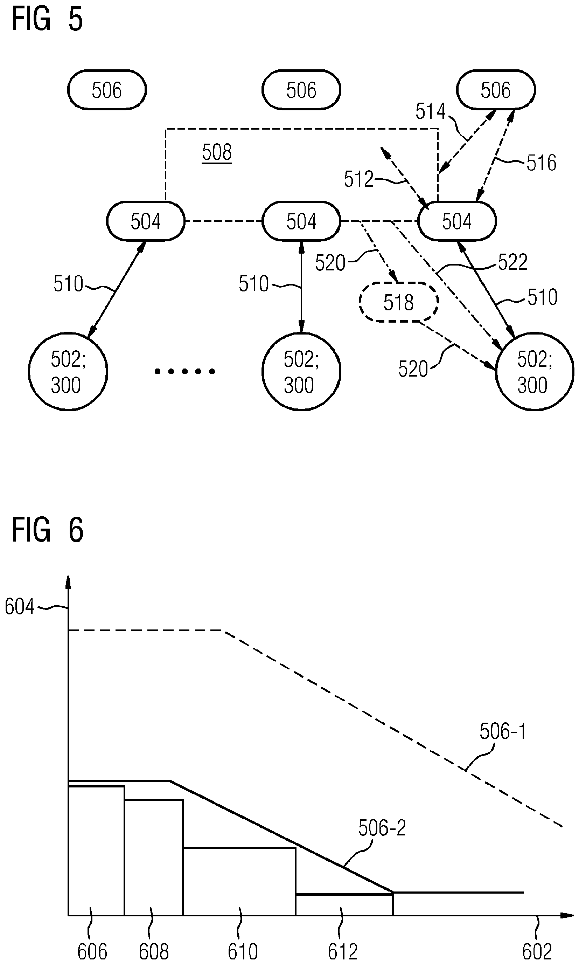

Das beispielhafte System in Fig. 5 umfasst eine Mehrzahl an technischen Komponenten 502 und/oder an medizinischen Feldgeräten, die jeweils einen in einer Verarbeitungseinheit 508 gespeicherten Digitalen Zwilling 504 der jeweiligen technischen Komponente 502 und/oder des jeweiligen medizinischen Feldgeräts umfassen.

Die Verarbeitungseinheit 508 kann gemäß einem lokalen Ausführungsbeispiel die Recheneinheit 204 der Vorrichtung 200 umfassen. Alternativ oder ergänzend kann die Verarbeitungseinheit 508 in einem zumindest teilweise zentralisierten System eine Zentrale Verarbeitungseinheit (englisch: "Central Processing Unit", kurz: CPU) umfassen, beispielsweise an einem Standort, der mit einer Mehrzahl technischer Komponenten 502 über Vorrichtungen 200 an unterschiedlichen medizinischen Standorten in Datenverbindung bringbar ist. Weiterhin alternativ oder ergänzend kann die Verarbeitungseinheit 508 virtualisiert sein und/oder in einer Cloud betrieben werden.

Der Digitale Zwilling 504 kann auch als Simulationsmodell bezeichnet werden. Alternativ oder ergänzend kann der Digitale Zwilling 504 jede Bewegung und/oder Belastung der jeweiligen technischen Komponente 502 und/oder des medizinischen Feldgeräts (beispielsweise virtuell) ausführen. Beispielsweise können Sensordaten an Bezugszeichen 510 zwischen der jeweiligen technischen Komponente 502 (beispielsweise mittels der Datenschnittstelle 202 der Vorrichtung 200) und dem Digitalen Zwilling 504 ausgetauscht werden. Die Sensordaten (auch: "Kenngrößen") können eine Position, eine Geschwindigkeit, eine Beschleunigung, ein Gewicht (beispielsweise eines Patienten), Bewegungsdaten, eine Temperatur und/oder eine Strahlendosis der jeweiligen technischen Komponente 502 des medizinischen Feldgeräts umfassen. Die Sensordaten können sich alternativ oder ergänzend auf eine oder mehrere technische Komponenten 502 des medizinischen Feldgeräts beziehen.

Beispielsweise kann die technische Komponente 502 des medizinischen Feldgeräts eine Patientenliege 300 gemäß Fig. 3 oder Fig. 4 umfassen und/oder von dieser umfasst sein. Im Ausführungsbeispiel der Patientenliege 300 der Fig. 3 kann die technische Komponente 502 beispielsweise das Kopfende 302, die Rumpfauflagefläche 304, die horizontale Auflagefläche 306 und/oder die Beinauflageflächen 308 umfassen.

Die Sensordaten werden über die Datenverbindung 510 an den Digitalen Zwilling 504 der technischen Komponente 502 und/oder des medizinischen Feldgeräts weitergeleitet. Die durch die Bewegung und/oder Verwendung einer (und/oder jeder) technischen Komponente 502 des medizinischen Feldgeräts entstandenen Belastungen können im Digitalen Zwillinge 504 (beispielsweise jeweils) berechnet werden.

Die berechnete Belastung kann an ein der technischen Komponente 502 des medizinischen Feldgeräts zugeordneten Betriebssicherheitsmodell (auch: "Betriebsfestigkeitsmodell") 506 übertragen werden. Alternativ oder ergänzend kann das Betriebssicherheitsmodell 506 mehrere oder alle technische Komponenten 502 des medizinischen Feldgeräts umfassen.

Im Fall einer Zentralen Datenverarbeitungsanlage 508 können Daten (beispielsweise die berechnete Belastung) vom Digitalen Zwilling 504 an Bezugszeichen 512 mit der Zentralen Datenverarbeitungsanlage 508 ausgetauscht werden. Die Zentrale Datenverarbeitungsanlage 508 kann bei Bezugszeichen 514 in Datenaustausch mit dem Betriebssicherheitsmodell 506 stehen oder gebracht werden. Alternativ oder ergänzend können Daten bei Bezugszeichen 516 zwischen dem Digitalen Zwilling 504 und dem Betriebssicherheitsmodell 506 (beispielsweise direkt und/oder bei einem zumindest teilweise lokalisierten Ausführungsbeispiel der Vorrichtung 200) ausgetauscht werden.

Das Betriebssicherheitsmodell 506 umfasst im Ausführungsbeispiel der Fig. 5 einen Betriebssicherheitszeitberechnungsalgorithmus, mittels dem die Restbetriebslaufzeit der technischen Komponente 502 und/oder des medizinischen Feldgeräts bestimmt wird. Die Restbetriebslaufzeit kann über die Datenverbindung 516, oder mittels der Zentralen Datenverarbeitungsanlage 514 über die Datenverbindungen 514; 512 an den Digitalen Zwilling 504 übertragen werden.

Gemäß einem Ausführungsbeispiel informiert der Digitale Zwilling 504 über die Datenverbindung 510 und/oder die Zentrale Datenverarbeitungsanlage 508 über die Datenverbindung 522; 520, optional mit einer zwischengeschalteten externen Serviceeinheit 518 (die auch als, insbesondere externe, Steuerung bezeichnet werden kann), das medizinische Feldgerät und/oder eine technische Komponente 502, und/oder die lokale Recheneinheit 204 und/oder Vorrichtung 200 über einen bevorstehenden Ablauf der Restbetriebslaufzeit.

Die Information über den bevorstehenden Ablauf der Restbetriebslaufzeit kann das Erzeugen S110 mindestens eines Steuersignals zum Steuern des medizinischen Feldgerätes umfassen. Das mindestens eine Steuersignal kann den Betrieb des medizinischen Feldgerätes (beispielsweise wahlweise) einschränken, vollständig sperren oder mit mindestens einer veränderten Steuergröße fortsetzen. Beispielsweise kann eine maximale Strahlendosis und/oder eine maximale Strahlenintensität eines Röntgengeräts reduziert und/oder eine Geschwindigkeit und/oder Beschleunigung einer Bewegung, beispielsweise einer Patientenliege 300 oder eines Deckenstativs 402, reduziert werden. Die Reduktion kann sich jeweils auf einen Normalbetrieb während der Betriebslaufzeit des medizinischen Feldgeräts und/oder der technischen Komponente 502 des medizinischen Feldgeräts beziehen.

Alternativ oder ergänzend können die Digitalen Zwillinge 504 und/oder Betriebssicherheitsmodelle 506 zumindest teilweise lokal ausgebildet sein, beispielsweise an einer Datenverarbeitungsstelle eines medizinischen Standorts und/oder an einer Vorrichtung 200, die benachbart zu dem medizinischen Feldgerät angeordnet ist und/oder in das medizinische Feldgerät (beispielsweise in eine Zentrale Datenverarbeitungseinheit, CPU, und/oder Hardware des medizinischen Feldgeräts) integriert ist.

Die Information über den bevorstehenden Ablauf der Restbetriebslaufzeit kann eine Meldung an eine externe Serviceeinheit 518 (auch: Steuerung) umfassen. Die externe Serviceeinheit 518 kann extern bezüglich der Vorrichtung 200 und/oder des medizinischen Feldgeräts sein. Beispielsweise kann die externe Serviceeinheit 518 eine technische Einheit eines medizinischen Standorts, und/oder technisches Personal der technischen Einheit, umfassen.

Alternativ oder ergänzend kann die externe Serviceeinheit 518 außerhalb des medizinischen Standorts des medizinischen Feldgeräts angeordnet sein. Beispielsweise kann die externe Serviceeinheit 518 eine technische Einheit und/oder technisches Personal umfassen, das für eine Vielzahl medizinischer Standorte zuständig ist.

Weiterhin alternativ oder ergänzend können der Digitale Zwilling 504 und das Betriebssicherheitsmodell 506 in einer Einheit angeordnet werden, beispielsweise lokal in einer Vorrichtung 200 umfassend eine Recheneinheit 204 und einen Datenspeicher 206. Die lokale Anordnung des Digitalen Zwillings 504 und des Betriebssicherheitsmodells 506 in der Vorrichtung 200, insbesondere am und/oder im medizinischen Feldgerät, kann auch als "schlanker Digitaler Zwilling" bezeichnet werden.

Das Betriebssicherheitsmodell 506 kann beispielsweise eine in einem Datenspeicher, beispielsweise dem Datenspeicher 206, hinterlegte Kennlinie und/oder eine Umsetzungstabelle (auch: "Lookup-Tabelle") umfassen, mittels der die Restbetriebslaufzeit der technischen Komponente 502 und/oder des medizinischen Feldgeräts bestimmt (beispielsweise abgelesen und/oder berechnet) werden kann.

Fig. 6 zeigt ein Beispiel einer Bestimmung einer Restbetriebslaufzeit. An Bezugszeichen 602 werden Anzahlen von Belastungen (und/oder Betriebsdauern der Belastungen) einer technischen Komponente 502 und/oder des medizinischen Feldgeräts je nach Höhe 604 der jeweiligen Belastung aufgetragen. In Fig. 6 sind die Höhen der Belastungen nach Klassen (auch: "Bins") 606; 608; 610; 612 von links nach rechts absteigend aufgetragen.

Bei Bezugszeichen 506-1 ist beispielhaft eine Kennlinie eines ersten Betriebssicherheitsmodells gezeigt. Bei Bezugszeichen 506-2 ist beispielhaft eine Kennlinie eines zweiten Betriebssicherheitsmodells gezeigt.

Die erste Kennlinie 506-1 kann ein Betriebssicherheitsmodell für eine technische Komponente 502 und/oder ein medizinisches Feldgerät umfassend eine erste Materialart sein. Beispielsweise kann die erste Materialart eine konservative Bauart und/oder eine hohe Belastbarkeit der technischen Komponente 502 und/oder des medizinischen Feldgeräts sein. Alternativ oder ergänzend kann die erste Materialart hohe Materialkosten repräsentieren.

Die erste Kennlinie 506-1 kann einer herkömmlichen technischen Komponente 502 und/oder einem herkömmlichen medizinischen Feldgerät entsprechen.

Beispielsweise kann ein medizinisches Feldgerät, insbesondere eine Patientenliege 300, mit zwanzig (20) Patienten mit einem maximalen Patientengewicht ausgesetzt sein an einer maximalen Anzahl von 250 Arbeitstagen pro Jahr. Eine Betriebszeit von zwanzig (20) Jahren kann einer maximalen Belastung des medizinischen Feldgeräts, beispielsweise der Patientenliege 300, mit einem Wert von 20·250·20=100.000=10⁵ zugeordnet sein. Der Wert 100.000=10⁵ kann einer maximal erlaubten Patientenlast und/oder einem "Worst Case" Szenario (beispielsweise, wenn alle Patienten das maximale Patientengewicht haben) entsprechen. In diesem Ausführungsbeispiel kann die Restbetriebslaufzeit nach zwanzig (20) Jahren abgelaufen sein.

Die zweite Kennlinie 506-2 kann ein Betriebssicherheitsmodell für eine technische Komponente 502 und/oder ein medizinisches Feldgerät umfassend eine zweite Materialart umfassen. Beispielsweise kann die zweite Materialart eine Leichtbauweise und/oder geringe Materialkosten umfassen.

Alternativ oder ergänzend kann die zweite Kennlinie 506-2 für ein Betriebssicherheitsmodell gelten, in dem nicht immer die maximale Belastung der technischen Komponente 502 und/oder des medizinischen Feldgeräts erreicht wird. Beispielsweise kann die Kennlinie zwanzig (20) Patienten pro Tag umfassen, von denen zwanzig Prozent (20%) das maximale Patientengewicht, zwanzig Prozent (20%) drei Viertel des maximalen Patientengewichts, dreißig Prozent (30%) die Hälfte des maximalen Patientengewichts und dreißig Prozent (30%) ein Viertel des maximalen Patientengewichts haben. Die Höhe des Patientengewichts kann beispielsweise den Klassen 606; 608; 610; 612 der Fig. 6 entsprechen.

Alternativ oder ergänzend kann die technische Komponente 502 und/oder das medizinische Feldgerät an weniger als der maximal möglichen Anzahl von Arbeitstagen pro Jahr belastet sein, beispielsweise an 150 Arbeitstagen pro Jahr.

Die geplante Betriebszeit kann zwanzig (20) Jahre betragen.

Ein Abgleich der Kennlinie 506-1 oder 506-2 kann beispielsweise wie anhand von Fig. 5 beschrieben erfolgen mittels eines Digitalen Zwillings 504, eines Betriebssicherheitsmodells 506 und einer optionalen externen Serviceeinheit 518. Aufgrund des Abgleichs der Kennlinie 506-1 oder 506-2 der Fig. 6 kann ein Steuersignal gemäß Verfahrensschritt S110 erzeugt werden. Alternativ oder ergänzend kann die externe Serviceeinheit 518 über die verbleibende Restbetriebslaufzeit informiert und ein Austausch der technischen Komponente 502 und/oder des medizinischen Feldgeräts geplant werden.

Grundlage zu Beginn für zukünftige Auslegungskriterien einer technischen Komponente 502 und/oder eines medizinischen Feldgeräts, beispielsweise hinsichtlich einer Wahl einer oder mehrerer Materialarten, bezüglich Kenngrößen einer Belastung, insbesondere Patientengewicht (beispielsweise für eine Patientenliege 300), Arbeitstage pro Jahr und Betriebsjahre können die herkömmlicherweise vorhandenen Erfahrungen und/oder auszuwertenden Daten bezüglich bereits erfolgter und/oder geplanter Systembelastungen sein.

Im Laufe der Betriebszeit der technischen Komponente 502 und/oder des medizinischen Feldgeräts kann zunehmend durch die Überwachung der Systembewegungen und Belastungen eine breitere und zunehmend optimierte Datenbasis für die optimalen Auslegungskriterien aufgebaut werden.

Somit kann eine Betriebssicherheit über eine zeitlich veränderliche Betriebsdauer des medizinischen Feldgeräts und/oder der technischen Komponente 502 erreicht werden. Alternativ oder ergänzend kann ein Einsatz von Materialien, beispielsweise zur Herstellung der technischen Komponente 502, optimiert werden.

Mittels der Bestimmung und Durchsetzung einer Betriebssicherheit einer technischen Komponente 502 eines medizinischen Feldgeräts in Abhängigkeit von über eine Gesamtbetriebszeit der technischen Komponente mittels mindestens eines Sensors überwachten Sensordaten des medizinischen Feldgeräts (auch: "Realtime Tracking") und/oder die Aktualisierung der Restbetriebszeit von medizinischen Komponenten und/oder Systemen mittels eines oder mehreren Digitalen Zwillings/en kann somit eine Dimensionierung für eine technische Komponente, wie einen Motor, ein Getriebe, und/oder Spindeln auf angepasste Lastzyklen kollektiv, und/oder eine längere Betriebslaufzeit ohne komplette Überholung der Komponenten und/oder Systeme, erreicht werden.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für Patientenliegen angewendet werden kann, sondern auch für andere medizinische Feldgeräte und deren technische Komponenten. Des Weiteren können die Bauteile der Vorrichtung, beispielsweise die Recheneinheit 204 und der Datenspeicher 206, auf mehreren physikalischen Produkten verteilt realisiert sein.

## Patentansprüche

1. Verfahren (100) zur Bestimmung und Durchsetzung einer Betriebssicherheit einer technischen Komponente (502) eines medizinischen Feldgeräts in Abhängigkeit von über eine Gesamtbetriebszeit der technischen Komponente (502) mittels mindestens eines Sensors überwachten Sensordaten des medizinischen Feldgeräts, umfassend folgende Verfahrensschritte:
- Empfangen (S102) von Sensordaten mittels des mindestens einen Sensors während einer Betriebszeit der technischen Komponente (502) des medizinischen Feldgeräts;
- Speichern (S104) der mittels des mindestens einen Sensors empfangenen Sensordaten in einem Datenspeicher (206);
- Zugreifen (S106) auf eine Verarbeitungseinheit (200; 508), in der ein Digitaler Zwilling (504) und/oder ein Betriebssicherheitsmodell (506) gespeichert ist, wobei das Zugreifen (S106) mit den empfangenen Sensordaten erfolgt, um auf Basis eines in der Verarbeitungseinheit (200; 508) gespeicherten Betriebssicherheitszeitberechnungsalgorithmus eine Restbetriebslaufzeit der technischen Komponente (502) zu berechnen;
- Ausgeben (S108) der berechneten Restbetriebslaufzeit der technischen Komponente (502) als Zwischenergebnis; und
- Erzeugen (S110) mindestens eines Steuersignals zum Steuern des medizinischen Feldgerätes auf Basis der berechneten Restbetriebslaufzeit, um den Betrieb des medizinischen Feldgerätes einzuschränken, vollständig zu sperren oder mit mindestens einer veränderten Steuergröße fortzusetzen.

2. Verfahren nach Patentanspruch 1, ferner umfassend den Verfahrensschritt:
Verarbeiten (S103) der Sensordaten, wobei das Verarbeiten (S103) ein Umwandeln der Sensordaten in ein vorbestimmtes Datenformat umfasst.

3. Verfahren nach einem der vorangehenden Patentansprüche, wobei das medizinische Feldgerät eine technische Komponente (502) umfasst, die ausgewählt ist aus der Gruppe bestehend aus:
- einer Patientenliege (300);
- einem Stativ (314; 402; 410), insbesondere einem Deckenstativ (402);
- einem C-Bogen; und
- einer Halbleiterbaugruppe.

4. Verfahren nach einem der vorangehenden Patentansprüche, wobei der zumindest eine Sensor ausgewählt ist aus der Gruppe bestehend aus:
- einem Positionssensor zur Erfassung von Positionsdaten (310; 312; 408; 604);
- einem Gewichtssensor zur Erfassung von Gewichtsdaten, mit dem die technische Komponente (502) belastet wird (604) ;
- einem Geschwindigkeitssensor zur Erfassung einer Geschwindigkeit (604);
- einem Beschleunigungssensor zur Erfassung einer Beschleunigung (604);
- einem Temperatursensor zur Erfassung einer Temperatur (604) ;
- einem Dosimeter zur Erfassung einer Strahlendosis (604) ;
- einem Zeitgeber zur Erfassung einer Betriebsdauer (602); und
- einem Zykluszähler zur Erfassung einer Anzahl Benutzungszyklen (602).

5. Verfahren nach einem der vorangehenden Patentansprüche, wobei das Erzeugen (S110) mindestens eines Steuersignals ferner ein Versenden einer Verschleißindikation an eine Steuerung, eine externe Serviceeinheit (518) und/oder an den Datenspeicher (206) umfasst.

6. Verfahren nach einem der vorangehenden Patentansprüche, wobei der Datenspeicher (206) und/oder die Verarbeitungseinheit (200; 508) an dem medizinischen Feldgerät, an einem medizinischen Standort, umfassend das medizinische Feldgerät und/oder an einem zentralen Standort, der zum Datenaustausch (510) mit einem oder mehreren medizinischen Standorten ausgebildet ist, angeordnet sind.

7. Verfahren nach einem der vorangehenden Patentansprüche, wobei in der Verarbeitungseinheit (200; 508) mindestens ein Referenzwert (506-1; 506-2) für die Sensordaten eines Sensors abgelegt ist, und/oder wobei der Digitale Zwilling (504) und/oder das Betriebssicherheitsmodell (506) mindestens einen Referenzwert (506-1; 506-2) für die Restbetriebslaufzeit in Abhängigkeit der Sensordaten des mindestens einen Sensors umfasst.

8. Vorrichtung (200) zur Bestimmung und Durchsetzung einer Betriebssicherheit einer technischen Komponente (502) eines medizinischen Feldgeräts in Abhängigkeit von über eine Gesamtbetriebszeit der technischen Komponente (502) mittels mindestens eines Sensors überwachten Sensordaten des medizinischen Feldgeräts, umfassend mindestens eine Recheneinheit (204), mit:
- Einer Empfangseinheit, die zum Empfangen von Sensordaten mittels des mindestens einen Sensors während einer Betriebszeit der technischen Komponente (502) des medizinischen Feldgeräts bestimmt ist;
- Einem Datenspeicher (206) zum Speichern der mittels des mindestens einen Sensors empfangenen Sensordaten;
- wobei die Recheneinheit (204) bestimmt ist zum Zugreifen auf eine Verarbeitungseinheit (200; 508), in der ein Digitaler Zwilling (504) und/oder ein Betriebssicherheitsmodell (506) gespeichert ist, wobei das Zugreifen mit den empfangenen Sensordaten erfolgt, um auf Basis eines in der Verarbeitungseinheit (200; 508) gespeicherten Betriebssicherheitszeitberechnungsalgorithmus eine Restbetriebslaufzeit der technischen Komponente (502) zu berechnen;
- Einer Ausgabeeinheit, die bestimmt ist zum Ausgeben der berechneten Restbetriebslaufzeit der technischen Komponente (502) als Zwischenergebnis; und
- wobei die Recheneinheit (204) bestimmt ist zum Erzeugen mindestens eines Steuersignals zum Steuern des medizinischen Feldgerätes auf Basis der berechneten Restbetriebslaufzeit, um den Betrieb des medizinischen Feldgerätes einzuschränken, vollständig zu sperren oder mit mindestens einer veränderten Steuergröße fortzusetzen.

9. Vorrichtung (200) nach Anspruch 8, die Mittel aufweist, um einen oder mehrere Schritte des Verfahrens gemäß Ansprüchen 2 bis 7 auszuführen.

10. System zur Bestimmung und Durchsetzung einer Betriebssicherheit einer technischen Komponente (502) eines medizinischen Feldgeräts in Abhängigkeit von über eine Gesamtbetriebszeit der technischen Komponente (502) mittels mindestens eines Sensors überwachten Sensordaten des medizinischen Feldgeräts, umfassend:
- mindestens eine Vorrichtung (200) und/oder eine lokale Recheneinheit (204), die dazu ausgebildet ist, das Verfahren nach einem der Ansprüche 1 bis 7 auszuführen, wobei die Vorrichtung (200) und/oder die lokale Recheneinheit (204) mindestens eine Datenschnittstelle (202) umfasst;
- mindestens einen Sensor zur Erfassung der Sensordaten, wobei der mindestens eine Sensor eine Datenschnittstelle zum Senden der Sensordaten an mindestens eine der mindestens einen Vorrichtung (200) und/oder lokalen Recheneinheit (204) umfasst; und
- einen Server, der einen Datenspeicher, eine Verarbeitungseinheit (508) und eine Datenschnittstelle (510; 512; 514; 520; 522) umfasst, wobei die Datenschnittstelle (510; 512; 514; 520; 522) zum Senden und Empfangen von Daten an die mindestens eine Vorrichtung (200) und/oder lokale Recheneinheit (204) ausgebildet ist, wobei der Datenspeicher zum Speichern der vom mindestens einen Sensor über die mindestens eine Vorrichtung (200) und/oder lokale Recheneinheit (204) empfangenen Sensordaten ausgebildet ist, und wobei in der Verarbeitungseinheit (508) ein Digitaler Zwilling (504) und/oder ein Betriebssicherheitsmodell (506) gespeichert ist, um auf Basis eines in der Verarbeitungseinheit (508) gespeicherten Betriebssicherheitszeitberechnungsalgorithmus eine Restbetriebslaufzeit der technischen Komponente (502) zu berechnen.

11. Computerprogrammprodukt, umfassend Befehle, die bei der Ausführung des Programms durch einen Computer diesen veranlassen, das Verfahren nach einem der Ansprüche 1 bis 7 auszuführen.
